# EUROPEAN PATENT SPECIFICATION

(11) **EP 2 581 067 B1**
(45) Date of publication and mention of the grant of the patent: **26.04.2017**
(21) Application number: 11792417.5
(22) Date of filing: 06.06.2011
(51) Int. Cl.: A61F 13/15, A61F 13/49, A61F 13/53, B29C 53/04, A61F 13/533

(54) **PROCESS FOR PRODUCTION OF ABSORBER**
VERFAHREN ZUR HERSTELLUNG EINES ABSORPTIONSMITTELS
PROCÉDÉ DE PRODUCTION D'UN PRODUIT ABSORBANT

(30) Priority: 10.06.2010 JP 2010132780
(43) Date of publication of application: 17.04.2013
(73) Proprietor: Kao Corporation, Chuo-Ku Tokyo 103-8210 (JP)
(72) Inventor: MARUYAMA, Hiroshi, Haga-gun Tochigi 321-3497 (JP); MOTEGI, Tomoyuki, Haga-gun Tochigi 321-3497 (JP); MATSUNAGA, Ryuji, Haga-gun Tochigi 321-3497 (JP); ONIZAWA, Yasuhiro, Haga-gun Tochigi 321-3497 (JP)
(74) Representative: Vossius & Partner Patentanwälte Rechtsanwälte mbB
(86) International application number: PCT/JP2011/062968
(87) International publication number: WO 2011/155460

(56) References cited:
- EP-A1- 1 666 010
- WO-A1-97/19659
- WO-A1-2010/015053
- GB-A- 1 493 882
- JP-A- 52 084 040
- JP-A- 2003 509 162
- JP-A- 2004 530 799
- JP-A- 2006 141 615
- JP-A- 2007 054 219
- JP-A- 2009 000 173
- JP-A- 2009 131 417

## Description

### Technical Field

The present invention relates to a method of producing an absorbent member.

### Background Art

In producing an absorbent article such as a disposal diaper, a sanitary napkin and an incontinence pad, raw material of absorbent member (fiber material such as defiberized pulp, particles of a superabsorbent polymer, and the like) which is fed along with an air stream is deposited as being suctioned to a recess portion formed at an outer circumferential face of a rotatable drum and a deposited aggregate which is deposited at the inside of the recess portion is used as an absorbent member as-is or as being covered with permeable sheet material.

Further, there have been known a technology to produce an absorbent member including a plurality of regions with mutually different basis weight of particles by arranging a porous region and non-porous region at a bottom face of a recess portion at which raw material of absorbent member is deposited and varying each amount of the particles to be deposited at both of the regions (see Patent Literature 1) and a technology to adjust a fiber-laminated amount in an absorbent member by arranging a plurality of regions at a bottom face of a recess portion at which raw material of absorbent member is deposited and varying suction force for each region (see Patent Literature 2).

Further, Patent Literature 3 discloses producing of an absorbent member in which a convex portion is formed on a porous bottom face plate forming a bottom face of a recess portion at which raw material of absorbent member is deposited and in which a portion corresponding to the convex portion becomes a lacking portion (non-fiber-laminated portion).

Further, Patent Literature 4 discloses an absorbent core molding drum in which a convex portion extended to be long in the circumferential direction of the drum is arranged at a bottom face of a molded recess portion at which raw material is deposited.

### Citation List

### Patent Literature

Patent Literature 1: JP 2009-232959 A
Patent Literature 2: JP 2004-222774 A
Patent Literature 3: JP 2008-206539 A
Patent Literature 4: US Patent Application Laid-Open No. 2006/105075A1

### Summary of Invention

### Technical Problem

In the technology of Patent Literature 1, the porous region and the non-porous region are formed at a single flat-plate-shaped support body with etching processing or punching processing. Accordingly, a fiber deposited amount is gradually varied at a boundary part between the porous region and the non-porous region.

Further, in the technology of Patent Literature 2 as well, since the plurality of regions to which different suction force is exerted is arranged on a single plate, the fiber deposited amount is gradually varied at a boundary part between adjacent regions. Accordingly, with the technologies of Cited Literatures 1 and 2, it is difficult to produce an absorbent member having a high density portion and a low density portion with large density difference.

Further, in Patent Literature 3, the purpose of arranging the convex portion on the bottom face plate is to produce the absorbent member having the lacking portion by preventing raw material from being deposited at a portion corresponding to the convex portion, as described above. Accordingly, in Patent Literature 3, there is no description to produce a deposited aggregate having a plurality of regions respectively with a different fiber deposited amount as depositing fiber on the convex portion and to produce an absorbent member having a high density portion and a low density portion with large density difference by utilizing the above.

Further, Patent Literature 4 discloses a structure in which a part of a convex portion is protruded from a circumferential face of a drum and a penetration hole is formed at the absorbent core to be produced so that density and basis weight are reduced via the convex portion by segmentalizing a mixture high density portion of the molded recess portion with the convex portion and a structure in which height is varied in part in a single convex portion as preferable structures. However, there is no description regarding specific teaching to form a high density portion and a low density portion with clearly different density.

Accordingly, the present invention related to a method of producing an absorbent member capable of effectively producing an absorbent member having a high density portion and a low density portion with clearly different density.

### Solution to Problem

According to an aspect of the present invention, there is provided a method of producing an absorbent member, including: a depositing step of depositing raw material of the absorbent member fed along with an air stream to a recess portion which is arranged at an outer circumferential face of a rotatable drum by suctioning; and a pressing step of pressing and compressing a deposited aggregate released from an inside of the recess portion, wherein the recess portion includes a suction portion which is formed of a porous plate to perform suctioning from a bottom face and a non-suction portion having an air-impermeable bottom face not to perform suctioning from the bottom face, while depth of the non-suction portion from the outer circumferential face of the rotatable drum is shallower than depth of the suction portion from the outer circumferential face of the rotatable drum; the deposited aggregate is obtained in the depositing step by depositing the raw material into the recess portion; and the absorbent member having a high density portion and a low density portion with mutually different density is obtained in the pressing step by pressing the deposited aggregate released from the recess portion. The low density portion is interposed between the high density portions whereby the high density portions are mutually separated in the absorbent member.

The present invention preferably includes following constituent elements.
(1) The absorbent member is an absorbent member in which a portion corresponding to the suction portion is the high density portion and a portion corresponding to the non-suction portion is the low density portion.
(2) The recess portion includes a plurality of suction portions each having a rectangular bottom face as the suction portion, and the suction portions are plurally formed in a circumferential direction and a width direction of the rotatable drum.
(3) A partition member including a plurality of first partition walls extended in the circumferential direction of the rotatable drum and a plurality of second partition walls extended in the width direction of the rotatable drum is arranged at the inside of the recess portion, and the bottom face of the non-suction portion is formed of the partition member.
(4) The first partition walls and the second partition walls are connected.
(5) The recess portion adopts a structure in which the suction portion and the non-suction portion are formed respectively into an elongated shape in the circumferential direction of the rotatable drum, the non-suction portions are plurally formed, and the suction portion and the non-suction portion are formed alternately in the width direction of the rotatable drum.
(6) The recess portion adopts a structure in which the suction portion and the non-suction portion are formed respectively into an elongated shape in the width direction of the rotatable drum, the non-suction portions are plurally formed, and the suction portion and the non-suction portion are formed alternately in the circumferential direction of the rotatable drum.
(7) The bottom face of the non-suction portion is formed of an air-impermeable member which is placed on the porous plate, and the air-impermeable member includes a side face portion having a linearly-shaped section in the thickness direction.
(8) The deposited aggregate is pressed in the pressing step between rolls each having a smooth surface or between a pair of emboss rolls in which a convex portion for embossing is provided to either roll or both rolls.
(9) The high density portion and the low density portion are pressed in the pressing step to have even apparent thickness.

### Advantageous Effects of Invention

According to the method of producing an absorbent member of the present invention, absorbent member having a high density portion and a low density portion with clearly different density can be effectively produced.

### Brief Description of Drawings

[Fig. 1] Fig. 1 is a schematic view illustrating an example of an apparatus of producing an absorbent member capable of being used for actualizing a method of producing an absorbent member of the present invention.
[Fig. 2] Fig. 2 is an exploded perspective view of an outer circumferential portion of a rotatable drum in the apparatus illustrated in Fig. 1.
[Fig. 3] Figs. 3(a) and 3(b) are views illustrating a part of the outer circumferential portion of the rotatable drum in the apparatus illustrated in Fig. 1 as Fig. 3(a) being a developed view of the outer circumferential portion and Fig. 3(b) being a sectional view at line III-III of Fig. 3(a).
[Fig. 4] Fig. 4 is a sectional view (corresponding view to Fig. 3(b)) illustrating a section of a recess portion at which fiber material is deposited.
[Fig. 5] Fig. 5(a) is a perspective view illustrating a deposited aggregate which is released from the recess portion of the apparatus illustrated in Fig. 1 and Fig. 5(b) is a sectional view at line V-V of the deposited aggregate.
[Fig. 6] Fig. 6(a) is a perspective view illustrating an example of an absorbent member obtained by performing pressure-compression against the deposited aggregate illustrated in Figs. 5(a) and 5(b) and Fig. 6(b) is a sectional view at line VI-VI of the absorbent member.
[Fig. 7] Fig. 7(a) is an explanatory view illustrating a diffusion direction control function of an absorbent member obtained in the present invention and Fig. 7(b) is a view illustrating a case with a conventional absorbent member without having the diffusion direction control function.

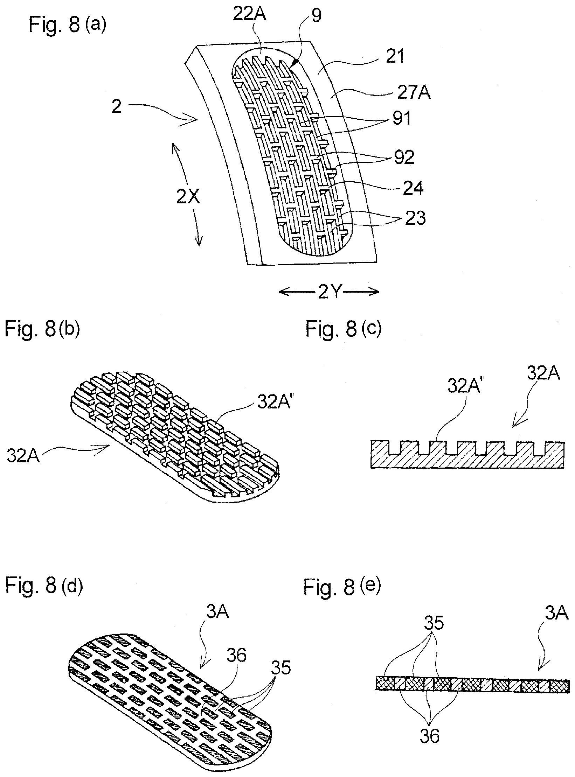

[Fig. 8] Figs. 8(a) to 8(e) are views illustrating another embodiment of the present invention. Fig. 8(a) is a perspective view illustrating a recess portion and a periphery thereof according to the embodiment, Fig. 8(b) is a perspective view illustrating a deposited aggregate released from the recess portion, Fig. 8(c) is a sectional view illustrating a section in the width direction at the center in the longitudinal direction of the deposited aggregate, Fig. 8(d) is a perspective view illustrating an absorbent member obtained by performing pressure-compression against the deposited aggregate illustrated in Fig. 8(b), and Fig. 8(e) is a sectional view illustrating a section in the width direction at the center in the longitudinal direction of the absorbent member.

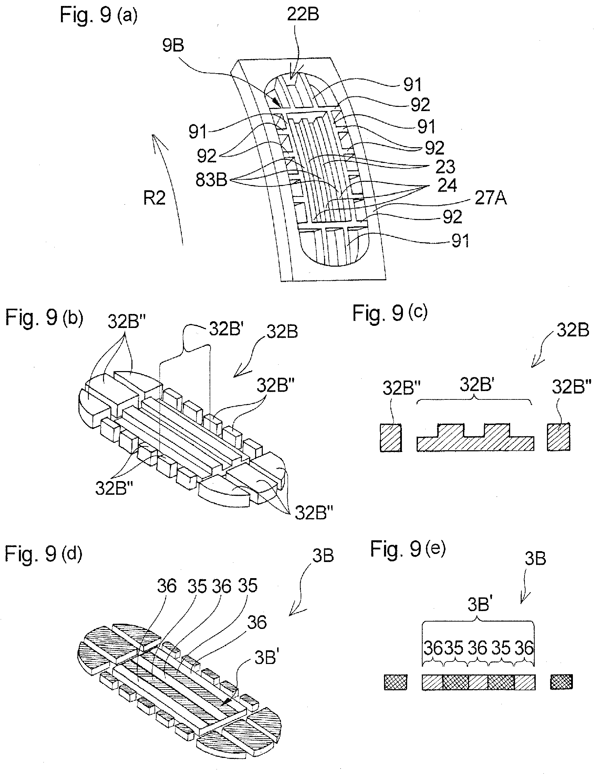

[Fig. 9] Figs. 9(a) to 9(e) are views illustrating still another embodiment of the present invention. Fig. 9(a) is a perspective view illustrating a recess portion and a periphery thereof according to the embodiment, Fig. 9(b) is a perspective view illustrating a deposited aggregate released from the recess portion, Fig. 9(c) is a sectional view illustrating a section in the width direction at the center in the longitudinal direction of the deposited aggregate, Fig. 9(d) is a perspective view illustrating an absorbent member obtained by performing pressure-compression against the deposited aggregate illustrated in Fig. 9(b), and Fig. 9(e) is a sectional view illustrating a section in the width direction at the center in the longitudinal direction of the absorbent member.
[Fig. 10] Fig. 10 is a view illustrating a part of an apparatus of producing an absorbent member used for another embodiment of the method of producing an absorbent member of the present invention.
[Fig. 11] Fig. 11 is a view illustrating another example of the absorbent member produced in the present invention being a corresponding view to Fig. 6(b).

### Description of Embodiments

In the following, the present invention will be described based on a preferable embodiment with reference to the drawings.

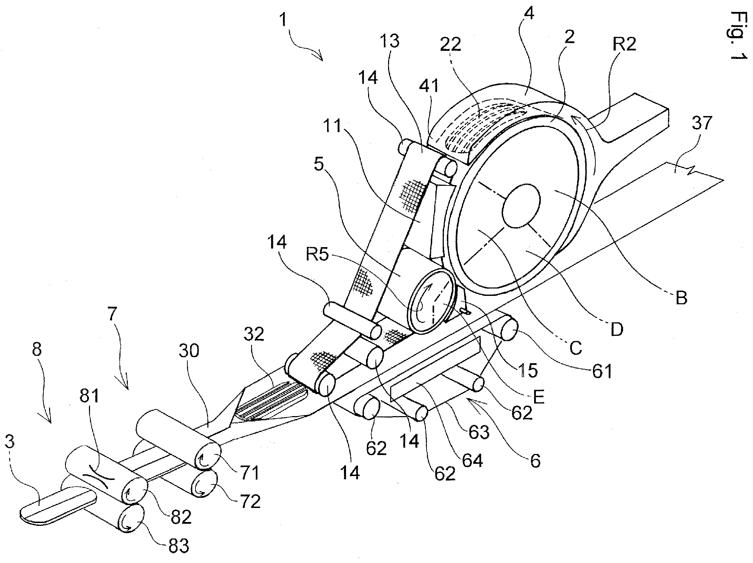

Fig. 1 illustrates an example of a producing apparatus capable of being used for actualizing a method of producing an absorbent member of the present invention.

An absorbent member producing apparatus 1 illustrated in Fig. 1 includes a rotatable drum 2 which is rotationally driven in a direction of arrow R2, a duct 4 which feeds fiber material being raw material of absorbent member to an outer circumferential face of the rotatable drum 2, a transfer roll 5 which is arranged obliquely below the rotatable drum 2 and is rotationally driven in a direction of arrow R5, a vacuum conveyer 6 which is arranged below the transfer roll 5, a pressing device 7, and a cutting device 8.

Further, in the producing apparatus 1, a vacuum box 11 is arranged between the duct 4 and the transfer roll 5 in the circumferential direction of the rotatable drum 2, a mesh belt 13 is arranged to pass between the vacuum box 11 and the rotatable drum 2 and between the transfer roll 5 and the rotatable drum 2, and an air screen plate 15 is arranged as being close to an outer circumferential face of the transfer roll 5.

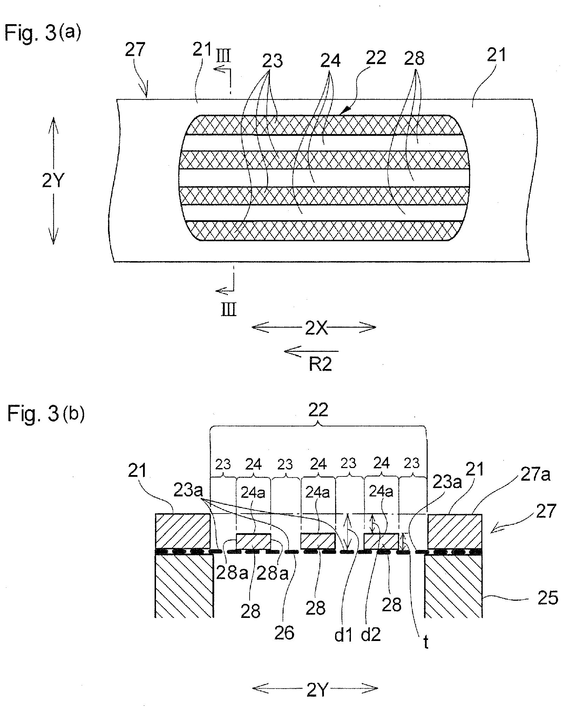

As illustrated in Fig. 1, the rotatable drum 2 is cylinder-shaped and is rotated about a horizontal axis as receiving power from a power source such as a motor. As illustrated in Fig. 3, the rotatable drum 2 is provided with a plurality of recess portions 22 at the outer circumferential face 21 thereof where fiber material being raw material of absorbent member is deposited. The recess portions 22 of the rotatable drum 2 are plurally formed at predetermined intervals in the circumferential direction (direction 2X) of the rotatable drum 2. In Fig. 3, direction 2X denotes the circumferential direction of the rotatable drum 2 and direction 2Y denotes a width direction of the rotatable drum 2 (a direction being in parallel to the rotational axis of the rotatable drum 2).

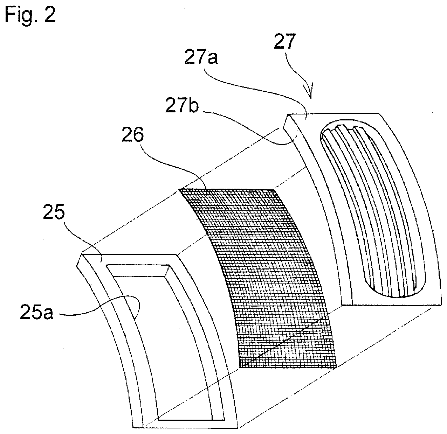

As illustrated in Figs. 2 and 3, the rotatable drum 2 includes a cylindrical frame body 25 which is made of a metal-made rigid body, a porous plate 26 which is fixed as being overlapped with the outer face side of the frame body 25, and a pattern forming plate 27 which is fixed as being overlapped with the outer face side of the porous plate 26. A variety of known fixing methods such as a bolt and an adhesive may be used without specific limitations as fixing means for the porous plate 26, the pattern forming plate 27 and the like.

The frame body 25 is shaped as making a ladder to be circular and connecting upper and lower ends thereof and is provided with communication holes 25a respectively at the corresponding recess portions 22 as penetrating inner and outer faces thereof.

The porous plate 26 has a number of pores and allows only air to pass as blocking fiber material being raw material of absorbent member which is fed along with an air stream. As the porous plate 26, anything which is conventionally used for such a type of fiber-laminating devices may be used without specific limitations. For example, it is possible to use, as the porous plate 26, a metal-made or resin-made mesh plate, a metal-made or resin-made plate having a number of pores formed with etching or punching, or the like.

The pattern forming plate 27 is provided with an outer face 27a which forms the outer circumferential face 21 of the rotatable drum 2 and an inner face 27b which is oriented to the rotational axis side of the rotatable drum 2 and includes a space having a shape corresponding to a solid shape in the recess portions 22 between the outer face 27a and the inner face 27b. As the pattern forming plate 27, anything which is conventionally used for such a type of fiber-laminating devices may be used without specific limitations. For example, it is possible to use, as the pattern forming plate 27, a plate at which an opening portion and a non-suction portion are formed by performing mechanical processing at a plate made of resin or metal such as stainless and aluminum, a plate at which an opening portion and a non-suction portion are formed as being integrated by molding, a plate to which a portion punched or etched is overlapped, or the like.

As illustrated in Fig. 3(b), the recess portion 22 is provided with a suction portion 23 which has a bottom face 23a formed of the porous plate 26 and which performs suctioning from the bottom face 23a while the recess portion 22 is passing over a later-mentioned space B, and a non-suction portion 24 which has an air-impermeable bottom 24a and which does not perform suctioning from bottom face 24a even when the recess portion 22 is passing over the later-mentioned space B.

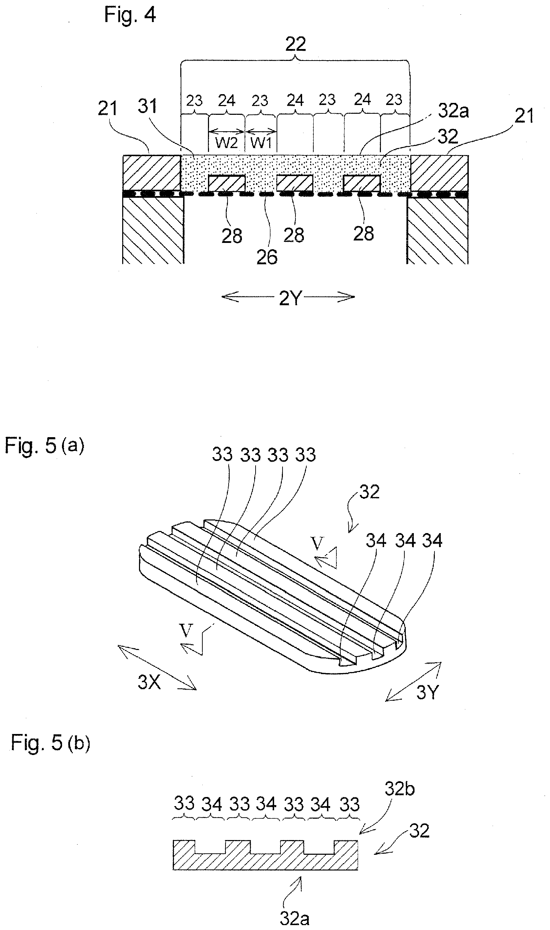

More specifically, as illustrated in Fig. 3(a), the suction portion 23 and the non-suction portion 24 are formed respectively into a shape being long in the circumferential direction (2X) of the rotatable drum. A plurality of the suction portions 23 and a plurality of the non-suction portions 24 are formed respectively. The non-suction portion 24 is linearly extended in the circumferential direction (2X) of the rotatable drum 2 and width W2 thereof (see Fig. 4) is constant in the circumferential direction (2X) of the rotatable drum 2. Further, the width W2 of the non-suction portions 24 is even in the depth direction of the recess portion 22. Further, the suction portion 23 and the non-suction portion 24 are formed alternately in the width direction (2Y) of the rotatable drum. Further, as illustrated in Fig. 3(b), the bottom face 24a of the non-suction portion 24 is formed of an air-impermeable member 28 which is arranged on the porous plate 26, so that suctioning from the bottom face 24a is not performed.

The recess portion 22 has different depth between the suction portion 23 and the non-suction portion 24 from the outer circumferential face 21 of the rotatable drum 2. The depth of the non-suction portion 24 from the outer circumferential face 21 of the rotatable drum 2 is shallower than the depth of the suction portion 23 from the outer circumferential face 21 of the rotatable drum 2. That is, as illustrated in Fig. 3(b), although the depth d1 of the suction portion 23 is the same as thickness of the pattern forming plate 27, the depth d2 of the non-suction portion 24 is the depth (d1-t) obtained by subtracting an amount of thickness t of the air-impermeable member 28 from the thickness of the pattern forming plate 27.

The depth d1 of the suction portion 23 and the depth d2 of the non-suction portion 24 are obtained by measuring distance from the outer circumferential face 21 of the rotatable drum 2 to the bottom faces 23a, 24a of the respective portions 23, 24 along a straight line perpendicular to the rotational axis (center line) of the rotatable drum 2. The thickness t of the air-impermeable member 28 is measured similarly.

In this manner, owing to that the suction portion 23 and the non-suction portion 24 having the air-impermeable member 28 are arranged at the recess portion 22 to which fiber material as raw material of absorbent member is deposited and that the depth d2 of the non-suction portion 24 is formed shallower than the depth d1 of the suction portion 23, it becomes easy to set large difference between an amount of fiber material deposited at the suction portion 23 and an amount thereof deposited at the non-suction portion 24 as depositing fiber material to both of the suction portion 23 and the non-suction portion 24.

At each recess portion 22, the thickness t of the air-impermeable member 28 is constant in the circumferential direction (2X) of the drum 2 and the depth d2 of the non-suction portion 24 is constant as well in the circumferential direction (2X) of the drum 2. More specifically, the air-impermeable member 28 has the thickness being constant at the entire range thereof and forms the flat bottom face 24a. Owing to that the depth d2 of the non-suction portion 24 is constant in the circumferential direction (2X) of the drum 2, an upper face 32a of the fiber material deposited at the recess portion 22 is more likely to be smooth over the entire range of the non-suction portion 24. From the similar viewpoint, it is preferable that the depth d2 of the non-suction portion 24 is constant as well in the width direction (2Y) of the drum 2. Further, in a case that a plurality of the non-suction portions 24 is arranged in the single recess portion 22, it is preferable that the depth d2 thereof is mutually the same.

Owing to that the upper face 32a of the fiber material deposited at the recess portion 22 is smooth, the amount of the fiber material at the suction portion and the non-suction portion becomes as designed. Accordingly, there is an advantage that targeted absorption performance can be obtained.

In an example illustrated in Figs. 2 and 3, the air-impermeable member 28 for forming the non-suction portion 24 structures a part of the pattern forming plate 27. More specifically, the air-impermeable member 28 having a rectangular section is integrally formed to form a face being flush with the inner face 27b of the pattern forming plate 27 made of synthetic resin. The air-impermeable member 28 in the present embodiment is indirectly fixed onto the porous plate 26 by fixing the pattern forming plate 27 integrated with the air-impermeable member 28 onto the frame body 25 and/or the porous plate 26. However, a lower face of the air-impermeable member 28 may be directly fixed onto the porous plate 26.

Spaces B, C and D which are mutually partitioned are formed at the inner side (rotational axis side) of the rotatable drum 2. A known exhaust device (not illustrated) such as an exhaust fan is connected to the space B. The inside of the space B can be maintained at negative pressure by operating the exhaust device. External air inflows to the space C with suctioning from the later-mentioned vacuum box 11 side. External air inflows to the space D with suctioning from the transfer roll 5 side. To favorably perform transfer above the space C (transfer of the deposited aggregate to the transfer roll and the like), the space C is partitioned from the space D which is to be a region of after-transferring. It is also possible to positively perform blowing from the space C toward the vacuum box 11. Here, one end of the rotatable drum 2 in the axial direction of the rotational axis is closed by a plate which is integrally rotated with the rotatable drum 2 and the other end is air-tightly closed by a non-rotating plate. Further, the spaces B to D are mutually partitioned by plates which are arranged from the rotational axis side of the rotatable drum 2 toward the inner face of the rotatable drum 2.

As illustrated in Fig. 1, one end side of the duct 4 covers the outer circumferential face of the rotatable drum 2 located above the space B and a fiber material introduction device is provided at the other end side (not illustrated). For example, the fiber material introduction device includes a grinder which grinds and makes sheet-shaped wood pulp into defiberized pulp and which feeds the defiberized pulp (fiber material) into the duct. It is also possible to arrange an absorbent polymer introduction portion which introduces particles of absorbent polymer at a midway of the duct 4.

Suctioning from the bottom face 23 a of the abovementioned suction portion 23 is performed while each recess portion 22 of the rotatable drum 2 passes over the space B which is maintained at negative pressure. Owing to the suctioning from the pores of the bottom faces 23a, there occurs an air stream in the duct 4 to convey raw material of absorbent member introduced from the fiber material introduction portion or the absorbent polymer introduction portion to the outer circumferential face of the rotatable drum 2. The conveyed raw material along with the air stream is deposited at the inside of the recess portion 22. The deposited raw material becomes the deposited aggregate 32.

The transfer roll 5 includes a air-permeable outer circumferential portion which is cylinder-shaped and the outer circumferential portion is rotated about a horizontal axis as receiving power from a power source such as a motor. A space E capable of decompressing the inside thereof is formed at a non-rotating portion at the inner side (rotational axis side) of the transfer roll 5. A known exhaust device (not illustrated) such as an exhaust fan is connected to the space E. The inside of the space E can be maintained at negative pressure by operating the exhaust device.

A number of suction holes which provide communication between the inside and outside is formed at an outer circumferential face 51 of the transfer roll 5. Air is suctioned from the outside to the inside while the suction holes pass over the space E which is maintained at negative pressure. Owing to the suction force, the deposited aggregate 32 in the recess portion 22 is smoothly transferred from an upper part of the rotatable drum 2 onto the transfer roll 5.

The vacuum conveyer 6 includes an endless air-permeable belt 63 which is looped over a drive roll 61 and driven rolls 62, 62, and a vacuum box 64 which is arranged at a position facing to the transfer roll 5 as sandwiching the air-permeable belt 63.

The vacuum box 11 having a box-like shape with upper and lower faces, both side faces and a back face includes an opening portion which is opened in a direction toward the rotatable drum 2. A known exhaust device (not illustrated) such as an intake fan is connected to the vacuum box 11 via an exhaust pipe (not illustrated) and the like. Owing to operation of the exhaust device, the inside of the vacuum box 11 can be maintained at negative pressure.

The mesh belt 13 is formed by connecting a belt-like air-permeable belt having meshes in an endless manner and is continuously moved along a predetermined route as being guided by a plurality of free rolls 14 and the transfer roll 5. The mesh belt 13 is driven with rotation of the transfer roll 5. As illustrated in Fig. 1, the mesh belt 13 is arranged to sequentially pass between the vacuum box 11 and the rotatable drum 2 and between the transfer roll 5 and the rotatable drum 2 after being introduced onto the outer circumferential face of the rotatable drum 2 at the vicinity of a downstream side end part 41 of the duct 4. The mesh belt 13 is contacted to the outer circumferential face of the rotatable drum 2 while passing before the opening portion of the vacuum box 11 and is moved onto the transfer roll 5 as being separated from the outer circumferential face of the rotatable drum 2 at the vicinity of the closest part between the transfer roll 5 and the rotatable drum 2.

The mesh belt 13 includes pores being smaller than the suction holes of the transfer roll 5. In accordance with suctioning from the suction holes of the transfer roll 5, suctioning is performed as well from the pores of the mesh belt 13 overlapped with the suction holes. The air screen plates 15 are arranged as a pair at both sides as sandwiching a region where the suction holes are formed in the width direction of the outer circumferential face of the transfer roll to prevent shape-losing and the like of the deposited aggregate 32 which is released from the recess portion 22 by preventing or suppressing wind inflow from a side. In particular, since wind inflow to a region having the suction holes of the transfer roll 5 from right-left outer sides in the width direction of the roll 5 is prevented or suppressed by the air screen plates 15, 15 arranged as being close to the roll 5, it is possible to effectively prevent inconvenience such that a deposited aggregate near both ends in the roll width direction leans to a deposited aggregate near the center and that arrangement of deposited aggregates is mutually disordered. Material of the air screen plates 15 is not specifically limited. In the light of providing stiffness to be capable of resisting wind, it is preferable to be made of metal or synthetic resin and to have thickness on the order of 0.5 to 10 mm.

The pressing device 7 includes a pair of rolls 71, 72 respectively having a smooth surface and is structured to be capable of compressing an object to be pressed which is introduced between the rolls 71, 72 as pressing from upper and lower faces thereof in a thickness direction. Instead of being provided with the pair of rolls 71, 72 with smooth surfaces, it is preferable to use a device including a pair of emboss rolls in which convex portions for embossing are formed at a circumferential face of either roll or both rolls from a viewpoint to be capable of improving absorption performance as forming a low density portion and a high density portion due to emboss at an absorbent member. Here, it is also possible to use a belt-conveyer type of a pressing device and the like instead of the pair of rolls.

A device which is conventionally used for cutting a continuous absorbent body in production of sanitary napkins or absorbent articles, or the like may be used as the cutting device 8 without specific limitations. The cutting device 8 illustrated in Fig. 1 includes a cutter roll 82 having a cutting blade 81 at a circumferential face thereof and an anvil roll 83 having a smooth circumferential face which receives the cutting blade.

Next, description is performed on a method to continuously produce absorbent members 3 by using the abovementioned absorbent member producing apparatus 1, that is, an embodiment of a method of producing an absorbent member of the present invention.

To produce the absorbent member 3 by using the absorbent member producing apparatus 1, the space B in the rotatable drum 2, the space E in the transfer roll 5 and the inside of the vacuum box 11 are set to be at negative pressure with operating exhaust devices connected respectively thereto. Owing to that the inside of the space B is set to be at negative pressure, an air stream to convey raw material of absorbent member to the outer circumferential face of the rotatable drum 2 occurs in the duct 4. Further, the rotatable drum 2 and the transfer roll 5 are rotated and the vacuum conveyer 6 is operated.

Subsequently, when fiber material is fed into the duct 4 with operating the fiber material introduction device, the fiber material is fed toward the outer circumferential face of the rotatable drum 2 in a form of dispersion along with the air stream flowing in the duct 4.

Fiber material 31 is suctioned and deposited at the recess portion 22 of the rotatable drum 2 while being conveyed through a region covered by the duct 4. Fig. 4 illustrates a section of the recess portion 22 at the time when passing through the downstream side end part 41 of the duct 4.

In the present embodiment, as illustrated in Fig. 4, the fiber material 31 is also deposited at the non-suction portion 24 where suctioning from the bottom face 24a is not performed as well as at the suction portion 23 where suctioning from the bottom face 23 a is performed. The fiber material is deposited only at the suction portion 23 at the upstream side of the duct 4. When height of the deposited fiber material reaches the thickness t of the air-impermeable member 28, the fiber material starts to be deposited on the air-impermeable member 28 as well in accordance with mutual entanglement of the fiber material and the air stream in the duct to convey the fiber material. The depositing is gradually advanced in a radial fashion from both side parts of the air-impermeable member 28 contacted to the suction portion and an upper face of the air-impermeable member 28 is to be covered with the fiber material. The recess portion is to be in a state of being completely covered with the fiber material at the downstream side of the duct 4.

Owing to that the suction force from the bottom face 23 a is gradually weakened in accordance with depositing of the fiber material 31, suction force difference between the upper faces of the non-suction portion 24 and the suction portion 23 becomes small. When the height of the fiber material deposited at the suction portion 23 reaches the thickness t of the air-impermeable member 28, even suction force is generated at the entire recess portion 22 and the fiber material becomes likely to be deposited also at the upper face of the air-impermeable member 28.

As illustrated in Fig. 4, it is preferable that depositing is performed so that a position of an upper face 32a of the fiber material deposited at each of the suction portion 23 and the non-suction portion 24 is approximately matched with a position of the outer circumferential face 21. In a case that density difference between the high density portion and the low density portion of the absorbent member to be formed is large (that the depth d2 of the non-suction portion 24 is shallow), long depositing time is required until the fiber material is sufficiently deposited at the non-suction portion 24. Therefore, it is preferable to design the part of the duct 4 which covers the rotatable drum to be long. In contrast, the depositing time may be short in a case that the density difference is small, it is preferable to design the length to be short.

Here, it is also possible to eliminate an excessive amount of fiber material with a scuffing roll or the like after the excessive amount of fiber material exceeding the position of the outer circumferential face 21 is deposited at the suction portion 23 and/or the non-suction portion 24.

In a case to vary a ratio between deposited amounts at the suction portion and the non-suction or to increase or decrease an amount of fiber to be deposited at the suction portion and the non-suction portion, width W2 of the non-suction portion 24 or a ratio (W1/W2) of width W1 of the suction portion 23 against the width W2 is adjusted or suction force generated at the bottom face 23a of the suction portion 23 is varied in accordance with the width W2, the ratio (W1/W2) or the like. Further, the depth d1 of the suction portion or a ratio (d2/d1) of the depth d2 of the non-suction portion 24 against the depth d1 of the suction portion 23 may be adjusted.

From viewpoints of releasability of the deposited aggregate 32 from the recess portion 22 and shape maintenance until a pressing step of the deposited aggregate 32 after releasing (in particular, a part 33 corresponding to the suction portion 23), the width W1 of the suction portion 23 is preferably 3 to 30 mm, and more preferably 5 to 20 mm. Further, from a viewpoint that the fiber material can be sufficiently deposited at the approximately same height of the outer circumferential face 21, the width W2 of the non-suction portion 24 is preferably 3 to 30 mm, and more preferably 5 to 20 mm. The ratio (W1/W2) between the width W1 of the suction portion 23 and the width W2 of the non-suction portion 24 is preferably 0.1 to 10, and more preferably 0.2 to 5.

Further, from viewpoints of releasability of the deposited aggregate 32 from the recess portion 22 and shape maintenance until the pressing step of the deposited aggregate 32 after releasing (in particular, the part 33 corresponding to the suction portion 23), the depth d1 of the suction portion 23 is preferably 1 to 30 mm, more preferably 3 to 20 mm, and even more preferably 5 to 10 mm.

Further, the ratio (d2/d1) of the depth d2 of the non-suction portion 24 against the depth d1 of the suction portion 23 is preferably 0.05 to 0.95, and more preferably 0.5 to 0.9.

Further, the ratio (width W2 / depth d2) between the width W2 and the depth d2 of the non-suction portion 24 is preferably 0.1 to 10, and more preferably 0.2 to 5.

Regarding the fiber material 31 to be deposited at the inside of the recess portion 22, basis weight of the suction portion 23 can be adjusted by the width W2 of the non-suction portion 24 and the thickness t of the air-impermeable member 28. For example, in a case that the depth d1 of the suction portion 23 is constant, the basis weight of the suction portion 23 can be increased without varying the depth d1 of the suction portion 23 by enlarging a value of the width W2 of the non-suction portion 24 or the thickness t of the air-impermeable member 28 and the basis weight of the suction portion 23 can be decreased by lessening the value of the width W2 of the non-suction portion 24 or the thickness t of the air-impermeable member 28. In this manner, owing to designing of the air-impermeable member 28, the basis weight of the suction portion 23 and the non-suction portion 24 can be adjusted and it becomes possible to easily produce an absorbent member which has desired flexibility and absorbing capability.

After the fiber material 31 is deposited at the suction portion 23 and the non-suction portion 24 in the recess portion 22 as described above, the rotatable drum 2 is further rotated. Subsequently, when the recess portion 22 reaches at an opposed position to the vacuum box 11, the deposited aggregate 32 in the recess portion 22 is in a state of being suctioned to the mesh belt 13 owing to suctioning from the vacuum box 11 and is conveyed to the closest part between the transfer roll 5 and the rotatable drum 2 or the vicinity thereof in the above state. Then, owing to suctioning from the transfer roll 5 side, the deposited aggregate 32 is transferred onto the transfer roll 5 along with the mesh belt 13 and is released from the inside of the recess portion 22 as well. As in the present embodiment, when the deposited aggregate 32 is conveyed as being suctioned from the side opposite to the rotatable drum 2 by the vacuum box 11 before being released from the recess portion 22 of the rotatable drum 2 and the deposited aggregate 32 is subsequently released from the recess portion 22, it is possible to effectively prevent occurrence of shape-losing of the deposited aggregate 32 and arrangement disordering of the deposited aggregate 32 when the deposited aggregate 32 is transferred onto the transfer roll 5 or other conveying means.

The deposited aggregate 32 transferred onto the transfer roll 5 is conveyed as being suctioned from the transfer roll 5 side and is transferred onto the vacuum conveyer 6.

In the present embodiment, as illustrated in Fig. 1, a core wrap sheet 37 made of tissue paper, permeable non-woven fabric or the like is introduced onto the vacuum conveyer 6 before the deposited aggregate 32 is placed and the deposited aggregate 32 is transferred onto the core wrap sheet 37.

Then, both side parts of the core wrap sheet 37 is folded at the downstream side thereof and both of upper and lower faces of the deposited aggregate 32 are covered with the core wrap sheet 37.

Subsequently, the deposited aggregate 32 in a state of being covered with the core wrap sheet 37 is compressed in the thickness direction as being introduced between the pair of rolls 71, 72 of the pressing device 7.

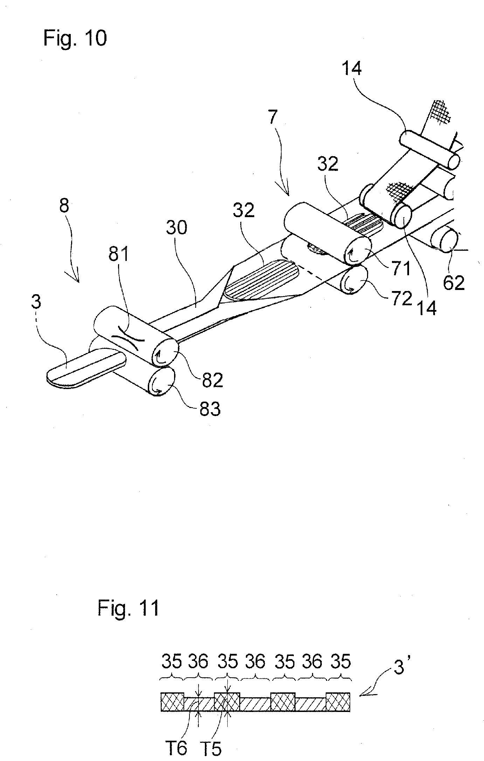

As illustrated in Fig. 10, pressure-compression against the deposited aggregate 32 due to the pressing device 7 or the like can be performed also against the deposited aggregate 32 before folding the core wrap sheet 37. In this case, pressing after folding may be performed or may not be performed.

Owing to performing pressure-compression against the deposited aggregate 32 before folding the core wrap sheet 37, folding of the core wrap sheet 37 is stabilized and shape-losing of the deposited aggregate 32 becomes unlikely to occur.

As illustrated in Fig. 5, in the deposited aggregate 32 right after being released from the recess portion 22, a portion 33 corresponding to the suction portion 23 is thick and a portion 34 corresponding to the non-suction portion 24 is thin. Further, one face 32a of the deposited aggregate 32 is approximately flat and the other face 32b is formed to be a corrugated face with large roughness. As illustrated in Fig. 5, a convex portion and a groove portion are formed on the corrugate face 32b as being extended in a direction (direction 3X) corresponding to the circumferential direction of the rotatable drum.

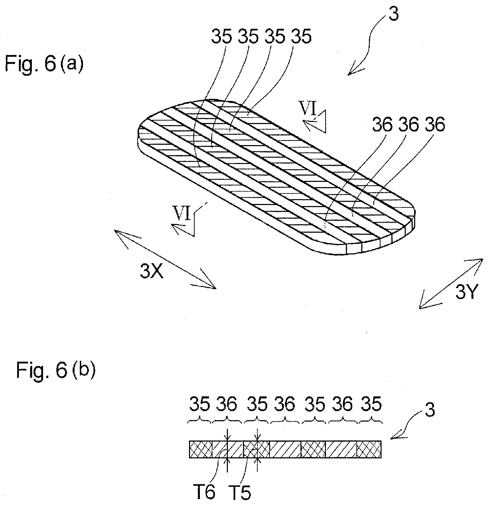

In the present embodiment, the deposited aggregate 32 in the above fashion is pressed by the pressing device 7 and thickness of the portion 33 corresponding to the suction portion 23 is actively decreased. Accordingly, thickness difference and/or a thickness ratio between the both portions 33, 34 are decreased to enable to obtain the absorbent member 3 illustrated in Fig. 6 in which the portion 33 corresponding to the suction portion 23 becomes the high density portion 35 and the portion 34 corresponding to the non-suction portion 24 becomes the low density portion 36. Pressing due to the pressing device 7 is preferably performed so that apparent thickness of the high density portion 35 and the low density portion 36 is to be even. In the absorbent member 3 illustrated in Fig. 6, the high density portion 35 and the low density portion 36 have even apparent thickness. The apparent thickness denotes thickness of a section of the absorbent member 3 without being loaded which can be observed with a microscope or the like. As illustrated in Fig. 6, in the absorbent member 3 obtained in the present embodiment, the high density portion 35 and the low density portion 36 are formed as being extended respectively in the direction (direction 3X) corresponding to the circumferential direction of the rotatable drum. The plural high density portions 35 are mutually separated via the low density portion 36 and the plural low density portions 36 are mutually separated via the high density portion 35. In a case that particles of absorbent polymer is introduced along with the fiber material 31 as raw material of absorbent member, the particles of absorbent polymer deposited at the high density portion 35 is higher in density compared to that at the low density portion 36.

During the pressure-compression due to the pressing device 7, either or both of the rolls 71, 72 may be heated or may not be heated. In a case that thermoplastic material is included in the absorbent member material, heating is preferably performed. Further, in a case that thermoplastic material is included in the absorbent member material, the pressing step may be performed with an ultrasonic device.

According to a method of producing an absorbent member of the present embodiment, it is possible to effectively produce the absorbent member 3 which has the high density portion 35 and the low density portion 36 with clearly different density, as described above. The absorbent member 3 preferably has the approximately same thickness at the high density portion 35 and the low density portion 36. From viewpoints of effectively developing a later-mentioned diffusion direction control function by enlarging density difference between the high density portion 35 and the low density portion 36, preventing bringing a feeling of strangeness to a user of an absorbent article when the absorbent member 3 is assembled into the absorbent article such as a sanitary napkin and a disposable diaper, and the like, a ratio (T5/T6) between thickness T5 of the high density portion 35 and thickness T6 of the low density portion 36 is preferably 1.0 to 1.2, and more preferably 1.0 to 1.1.

Regarding the thickness T5 of the high density portion 35 and the thickness T6 of the low density portion 36 described here, the absorbent member 3 is cut so as not press the thickness thereof to the extent possible and thickness of the high density portion 35 and the low density portion 36 is respectively measured without being loaded as enlarging the cut face with a microscope or the like.

Further, owing to that the opening portion of the pattern forming plate forms the high density portion and the non-opening portion thereof forms the low density portion without having the porous plate being solid-shaped, forming of the non-opening portion can support various widths and shapes and a complicated absorbent member can be processed. Further, from a viewpoint of providing clear density difference between the high density portion and the low density portion, it is preferable that a section of the air-impermeable member 28 along the thickness direction (direction d1 in Fig. 3(b)) has linear side face portions 28a, 28a, and in particular, has a sectional rectangular shape as illustrated in Fig. 3(b).

For example, as an absorbent member 3' illustrated in Fig. 11, an absorbent member produced in the present invention may have different thickness between the high density portion 35 and the low density portion 36. In this case, it is preferable that the thickness T6 of the low density portion 36 is smaller than the thickness T5 of the high density portion 35. Owing to small thickness of the low density portion 36, the low density portion forms a flexion crease which is likely to be bent and absorbent member flexibility is improved.

According to the method of producing an absorbent member of the present embodiment, it is possible to easily produce an absorbent member having the high density portion 35 and the low density portion 36 with clearly different density. Further, it is also easy to produce an absorbent member having clear density difference at a boundary part between the high density portion 35 and the low density portion 36.

Regarding an absorbent member produced in the present invention, for example, basis weight of the low density portion 36 is preferably 50 to 300 g/m², and more preferably 100 to 200 g/m². Basis weight of the high density portion 35 is preferably 200 to 800 g/m², and more preferably 300 to 500 g/m². The ratio between the density of the high density portion 35 and the density of the low density portion 36 (the former / the latter) is preferably 1.1 to 5.0, and more preferably 1.5 to 3.0. Preferable widths (widths in direction 3Y) of the high density portion 35 and the low density portion 36 and a ratio of the width are similar to the abovementioned preferable widths and ratio of the suction portion 23 and the non-suction portion 24.

### [Method of measuring density of high density portion and low density portion]

### [Measurement of thickness of measurement piece]

Thickness as an entire measurement piece including a high density portion and a low density portion is measured. Thickness measurement is based on JIS-P8118:1998. Here, measurement is performed with a peacock type accurate measuring instrument (Type R1-C) which is a micrometer having two parallel pressing faces (a fixed pressing face and a movable pressing face) as a diameter of the movable pressing face of a measuring head being 5 mm and the pressure being 2.0 N or lower. A test piece for measurement has a size being equal to or larger than a size of a below-mentioned plate. The plate (weight 5.4 g) having a size of 20 mm by 20 mm is placed on the test piece, the measuring head movable pressing face is operated at speed being 3 mm/s or slower to be abutted to the plate, and a value is read out right after being stabilized. The pressure between the pressing faces (pressure applied to the test piece) becomes 2 kPa or lower.

### [Measurement of density of respective high density portion and low density portion]

The density of the high density portion 35 and the low density portion 36 is measured based on JIS-P8118:1998. Specifically, calculation is performed as "D (density: g/cm³) = W (basis weight: g/m²) / T (thickness: mm)". Regarding basis weight as described in JIS-P8124, a test piece is cut with a cutting machine or a cutter and weight thereof is measured with a scale. Basis weight is calculated as dividing the measured weight by area. Thickness is measured with the abovementioned thickness measuring method as the entire measurement piece including the high density portion and the low density portion. In basis weight measurement of the present application, basis weight is calculated as measuring weight after cutting the high density portion and the low density portion to have specified area respectively to be matched with processing dimensions.

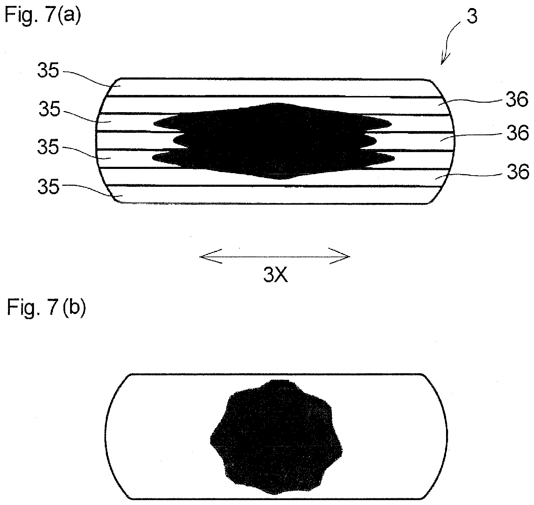

In the preferable absorbent member 3 produced in the present invention, owing to having the high density portion 35 and the low density portion 36 with clearly different density, the diffusion direction control function is effectively developed as illustrated in Fig. 7(a). Liquid such as menstrual blood and urine fed on the absorbent member 3 is quickly absorbed to the low density portion 36 and is favorably dispersed in an extending direction of the high density portion 35. Meanwhile, since the high density portions 35 are mutually separated via the low density portion 36, dispersion in a direction being perpendicular to the extending direction of the high density portion 35 is suppressed. The liquid dispersed along the high density portion 35 is reliably absorbed and held.

Accordingly, liquid leakage from both ends in the direction intersecting to the high density portion 35 is prevented as suppressing liquid dispersion in the direction and absorption capacity of the absorbent member can be effectively utilized in the extending direction of the high density portion 35.

For example, owing to that the extending direction of the high density portion 35 and the low density portion 36 is matched with a front-rear direction of a user when the absorbent member illustrated in Figs. 6 and 7(a) is assembled into an absorbent article such as a sanitary napkin and a disposable diaper, it is possible to obtain the absorbent article which has superior leakage preventing performance and utilizes absorption capacity of the absorbent member. Here, in an absorbent member produced in the present invention, the high density portion 35 and the low density portion 36 may be extended respectively in the width direction of the absorbent member. With such an absorbent member, owing to that a region having the high density portion 35 and the low density portion 36 in alternate order is arranged at the vicinity of the front end and/or the vicinity of the rear end in the front-rear direction of a user, it is possible to obtain an absorbent article having superior liquid-leakage preventing performance from the front-rear direction of the absorbent article.

Fig. 7(b) illustrates a liquid dispersion state in a case that the same amount of liquid as Fig. 7(a) is fed to a conventional absorbent member having even density over the entire region. With such an absorbent member, the liquid is dispersed to a similar extent in all directions. Accordingly, the liquid easily reaches both ends in the width direction having a small dimension and the liquid is likely to be leaked from the both ends.

In the embodiment illustrated in Fig. 1, compressing with the pressing device 7 and cutting with the cutting device 8 are performed after upper and lower faces of the deposited aggregate 32 which is released from the recess portion 22 are covered with the core wrap sheet 37. A belt-shaped body 30 having the deposited aggregate 32 placed intermittently in the flow direction is cut by the cutting device 8 at a position where the deposited aggregate 32 does not exist to have length of one piece of the absorbent article. Accordingly, in the absorbent member 3 after cutting with the cutting device 8, the upper and lower faces are covered with one sheet of the core wrap sheet 37. However, the absorbent member 3 produced in the present invention is not limited to that the upper and lower faces are covered with the single core wrap sheet 37. It is also possible that the upper and lower faces are covered separately by two core wrap sheets or that the upper and lower faces are not covered with a core wrap sheet.

Figs. 8 and 9 are explanatory views of another embodiments of the present invention.

Fig. 8(a) is a perspective view illustrating a recess portion 22A according to another embodiment. In the recess portion 22A, a partition member 9 having height not to reach the outer circumferential face 21 of the rotatable drum is arranged at the inside of a space having an oblong section formed by a pattern forming plate 27A. The partition member 9 includes a plurality of first partition walls 91 extending in the circumferential direction (2X) of the rotatable drum 2 and a plurality of second partition walls 92 which extends in the width direction (2Y) of the rotatable drum 2 and connects the first partition walls or the first partition wall and an inner circumferential face of the pattern forming plate 27A. The partition member 9 is an air-impermeable member which is made of synthetic resin or metal.

Owing to the partition member 9, a number of suction portions 23 where suctioning is performed respectively from a bottom face which is formed of a porous plate (not illustrated) and a non-suction portion 24 where suctioning is not performed from a bottom face which is formed of an upper end of the partition member 9 are formed in the recess portion 22A.

In the recess portion 22A illustrated in Fig. 8(a), the suction portions 23 whose bottom faces are rectangle-shaped are plurally formed in the circumferential direction (2X) and the width direction (2Y) of the rotatable drum 2. That is, a plurality of the suction portions 23 are intermittently arranged in the circumferential direction (2X) of the rotatable drum and a plurality of the suction portions 23 are intermittently arranged as well in the width direction (2Y) of the rotatable drum. The second partition wall 92 is located between the suction portions 23 in the circumferential direction (2X) of the rotatable drum 2 and the first partition wall 91 is located between the suction portions 23 in the width direction (2Y) of the rotatable drum. The bottom face of the suction portion 23 is formed of the porous plate (not illustrated).

Here, the rectangular shape of the bottom face of the suction portion denotes that a shape in a planar view viewing from the normal line direction of the outer circumferential face of the rotatable drum 2 is rectangular. The suction portions 23 illustrated in Fig. 8 are rectangle-shaped respectively in a planar view. Further, a bottom face of the suction portion 23 which is adjacent to an inner circumferential face of the recess portion 22A has an edge part along the shape of the inner circumferential face.

The partition member 9 illustrated in Fig. 8(a) includes the second partition walls 92 extended in the width direction (2Y) of the rotatable drum. Accordingly, fiber material fed toward the recess portion 22A is caught by the air-impermeable portion (second partition walls 92) extended in the width direction to be in a state of being easily deposited at the suction portion 23 by the thickness of the partition member 9 and being easily deposited on the partition member 9.

Further, in the partition member 9 illustrated in Fig. 8(a), the first partition walls 91 and the second partition walls 92 are connected. Accordingly, depositing of the fiber material and the like is more facilitated owing to being caught by the second partition walls 92.

Here, the suction portion 23 at a center region of the recess portion 22A is surrounded at the circumference thereof by the first partition walls 91 and the second partition walls 92 by the thickness t of the partition member 9 being the air-impermeable member. Further, in a planar view of the recess portion 22A, the suction portion 23 and the non-suction portion 24 are arranged like sea-island as the non-suction portion 24 being a sea and the suction portion 23 being an island.

A deposited aggregate 32A illustrated in Figs. 8(b) and 8(c) is a deposited aggregate obtained by depositing fiber material (defiberized pulp and the like) as raw material of absorbent member at the inside of the recess portion 22A. One face of the deposited aggregate 32A is flat and the other face thereof is formed to be a corrugated face on which a number of block-shaped convex portions 32A' is formed. An absorbent member 3A in which the high density portion 35 and the low density portion 36 are arranged as illustrated in Figs. 8(d) and 8(e) can be obtained by compressing the deposited aggregate 32A in the thickness direction as pressing with the pressing device. In the absorbent member 3A of Figs. 8(d) and 8(e), the high density portion 35 and the low density portion 36 have even apparent thickness. Further, the plural high density portions 35 are mutually separated via the low density portion 36 and the low density portion 36 is continuously formed in the planar direction of the absorbent member 3A.

In the recess portion 22A illustrated in Fig. 8(a), both of thickness of the partition member 9 and depth of the non-suction portion 24, of which the bottom face is formed of the partition member 9, from the drum outer circumferential face are constant in the circumferential direction (2X) of the drum 2 and the bottom face formed of the partition member 9 being the non-suction portion 24 is flat. Accordingly, an upper face of the fiber material deposited at the recess portion 22A is likely to be smooth. In addition, the thickness of the partition member 9 and the depth of the non-suction portion 24 from the drum outer circumferential face are constant also in the width direction (2Y) of the drum 2. Accordingly, the upper face of the fiber material deposited at the recess portion 22A is more likely to be smooth.

Fig. 9(a) is a perspective view illustrating a recess portion 22B of still another embodiment. In the recess portion 22B, a partition member 9B is arranged at the inside of a space having an oblong section formed by the pattern forming plate 27A. The partition member 9B includes first and second partition walls 91, 92 having height to reach the outer circumferential face 21 of the rotatable drum at the front, rear, right and left sides in the rotational direction R2 of the rotatable drum and three air-impermeable members 83B having height not to reach the outer circumferential face 21 of the rotatable drum at a center part thereof. Owing to the partition member 9B, a number of suction portions 23 where suctioning is performed respectively from a bottom face which is formed of a porous plate (not illustrated) and a non-suction portion 24 where suctioning is not performed from a bottom face which is formed of the air-impermeable member 83B are formed at the center part in the recess portion 22B. Here, it is also possible to have a deposited state in which fiber material is connected in whole as being deposited on the partition member 9B and the first and second partition walls 91, 92 as well by lowering the partition member 9B and the partition walls 91, 92 from the height to reach the outer circumferential face 21 of the rotatable drum.

A deposited aggregate 32B illustrated in Figs. 9(b) and 9(c) is a deposited aggregate obtained by depositing fiber material (defiberized pulp and the like) as raw material of absorbent member at the recess portion 22B. The deposited aggregate 32B includes, at center part thereof, a center deposited aggregate 32B' as one face being flat and the other face being a corrugated face and a plurality of peripheral deposited aggregates 32B" at a circumference of the center deposited aggregate 32B' which is separated from the center deposited aggregate 32B'. An absorbent member 3B in which the high density portion 35 and the low density portion 36 are arranged at the center part as illustrated in Figs. 9(d) and 9(e) can be obtained by compressing the deposited aggregate 32B in the thickness direction as pressing with the pressing device. In the absorbent member 3B, the two high density portions 35 are mutually separated via the low density portion 36 and the plural low density portions 36 are mutually separated via the high density portions 35. In the absorbent member 3B, the high density portions 35 and the low density portions 36 have even apparent thickness.

In the recess portion 22B illustrated in Fig. 9(a), depth of the non-suction portion 24 (depth from the drum outer circumferential face to the air-impermeable member 83B) is constant in the circumferential direction (2X) of the drum 2. Accordingly, an upper face of the fiber material deposited at the suction portion 23 and the non-suction portion 24 of the recess portion 22B is likely to be smooth. In addition, depth of the non-suction portion 24 (depth from the drum outer circumferential face to the air-impermeable member 83B) is constant also in the width direction (2Y) of the drum 2. Further, the bottom face formed of the air-impermeable member 83B is flat. Accordingly, the upper face of the fiber material deposited at the recess portion 22B, the suction portion 23 and the non-suction portion 24 is more likely to be smooth. In a case that the fiber material is deposited also on the partition walls 91, 92 of the partition member 9B, the depth of the parts thereof from the drum outer circumferential face is preferably to be constant from a viewpoint to smoothen a surface of the deposited aggregate.

Raw material of absorbent member includes fiber material.

A variety of material conventionally used for an absorbent member of an absorbent article such as a sanitary napkin, a panty liner and a disposal diaper may be used as the fiber material being raw material of absorbent member without specific limitations. Examples of the above include pulp fiber such as defiberized pulp, short fiber of cellulose-based fiber such as rayon fiber and cotton fiber, and short fiber of synthetic fiber such as polyethylene. The abovementioned fiber may be used in one kind singularly or in two or more kinds as being combined. Further, as raw material of the absorbent member 3, absorbent polymer may be introduced into the duct 4 along with fiber material. Further, as fiber-like raw material, fiber-like absorbent polymer may be used singularly or along with fiber material. Further, deodorant, antimicrobial and the like may be fed as needed along with fiber material and the like. In an absorbent member, basis weight of fiber material at a high density portion is preferably larger than basis weight of fiber material at a low density portion.

In the above, description is performed on several embodiments of the method of producing an absorbent member of the present invention. Not limited to the abovementioned embodiments, the present invention may be modified appropriately.

In the embodiment illustrated in Fig. 1, the deposited aggregate 32 in the recess portion is transferred onto the mesh belt 13 which is fed onto the transfer roll 5. Alternatively, the deposited aggregate 32 may be transferred onto the outer circumferential face of the transfer roll 5 onto which the mesh belt 13 is not fed, for example. Not just the mesh belt 13, it is also possible to omit the vacuum box 11, the air screen plate 15 and the like.

Further, the deposited aggregate 32 in the recess portion may be transferred directly onto the core wrap sheet 37 which is fed to the vacuum conveyer 6 without being assisted by the transfer roll 5. The deposited aggregate 32 may be transferred onto a belt conveyer without having an intake mechanism or onto other conveying means after being conveyed by the transfer roll 5.

Further, in the recess portion, the suction portion 23 and the non-suction portion 24 may be alternately formed respectively into a belt shape as being extended respectively into a shape elongated in the width direction of the rotatable drum 2. Further, each number of the suction portions 23 and the non-suction portions 24 which are belt-shaped may be 2 to 10, for example. The number of the suction portions 23 may be the same as or may be different from the number of the non-suction portions 24. The non-suction portion 24 may be curved in the circumferential direction (direction 2X) of the rotatable drum 2.

Further, the air-impermeable member 28 which structures the non-suction portion 24 may include a side face portion having a linearly-shaped section in the thickness direction. The air-impermeable member 28 may form a tapered shape at the suction portion 23 by enlarging distance between the side face portions of the adjacent air-impermeable members 28 or distance between the side face portion of the air-impermeable member 28 and the inner circumferential face of the recess portion as being apart from the bottom face. In this case, releasability of deposited raw material of an absorbent member (deposited aggregate) from the recess portion is improved.

Further, an absorbent member to be produced may include a region having belt-shaped high density portions and low density portions in a stripe-like fashion at a center part in the longitudinal direction or at the front side or rear side in the longitudinal direction and may be provided with an absorbent member at the front side and/or rear side of the region as being separated from the region. Further, an absorbent member to be produced may include a region having a belt-shaped high density portions and low density portions in a stripe-like fashion at a center part in the width direction and may be provided with an absorbent member at either or both of sides sandwiching the region as being separated from the region.

Further, the depth d2 of the non-suction portion 24 (i.e., thickness t of the air-impermeable member 28) may be varied among the center part, the front side and/or the rear side in the longitudinal direction along the circumferential direction of the rotatable drum of the single recess portion 22. For example, in the non-suction portion 24, the depth d2 at the center part in the longitudinal direction may be deeper than the depth d2 at the front side and/or the rear side.

The absorbent member produced in the present invention is preferably used as an absorbent member of an absorbent article. The absorbent article is mainly used for absorbing and retaining body fluid excreted from a body such as urine and menstrual blood. Examples of the absorbent article include a disposal diaper, a sanitary napkin, an incontinence pad, and a panty liner. However, not limited to the above, articles used for absorbing liquid excreted from a human body may be widely included.

Typically, an absorbent article includes a topsheet, a backsheet, and a liquid-retaining absorbent member which is interposed between the both sheets. In the absorbent member, upper and lower faces may be covered with one or plural core wrap sheets. The backsheet may have or may not have vapor permeability. Further, the absorbent article may include a variety of members in accordance with specific applications of the absorbent article. Such members are known to persons skilled in the art. For example, in a case that the absorbent article is applied to a disposal diaper or a sanitary napkin, a pair or two or more pairs of standing guards may be arranged at the outside of both raised side parts of the absorbent member.

An element whose description is skipped in one of the abovementioned embodiments and an element which is included in only one embodiment may be appropriately applied respectively to another embodiment. Further, elements in the respective embodiments may be mutually replaced appropriately among the embodiments.

## Claims

1. A method of producing an absorbent member, comprising:
a depositing step of depositing raw material of the absorbent member fed along with an air stream to a recess portion which is arranged at an outer circumferential face of a rotatable drum by suctioning; and
a pressing step of pressing and compressing a deposited aggregate released from an inside of the recess portion,
wherein the recess portion includes a suction portion which is formed of a porous plate to perform suctioning from a bottom face and a non-suction portion having an air-impermeable bottom face not to perform suctioning from the bottom face, while depth of the non-suction portion from the outer circumferential face of the rotatable drum is shallower than depth of the suction portion from the outer circumferential face of the rotatable drum;
the deposited aggregate is obtained in the depositing step by depositing the raw material into the recess portion; and
the absorbent member having a high density portion and a low density portion with mutually different density is obtained in the pressing step by pressing the deposited aggregate released from the recess portion;
wherein the low density portion is interposed between the high density portions whereby the high density portions are mutually separated in the absorbent member.

2. The method of producing an absorbent member according to claim 1,
wherein the absorbent member is an absorbent member in which a portion corresponding to the suction portion is the high density portion and a portion corresponding to the non-suction portion is the low density portion.

3. The method of producing an absorbent member according to claim 1 or claim 2,
wherein the recess portion includes a plurality of suction portions each having a rectangular bottom face as the suction portion; and
the suction portions are plurally formed in a circumferential direction and a width direction of the rotatable drum.

4. The method of producing an absorbent member according to claim 3,
wherein a partition member including a plurality of first partition walls extended in the circumferential direction of the rotatable drum and a plurality of second partition walls extended in the width direction of the rotatable drum is arranged at the inside of the recess portion; and
the bottom face of the non-suction portion is formed of the partition member.

5. The method of producing an absorbent member according to claim 4,
wherein the first partition walls and the second partition walls are connected.

6. The method of producing an absorbent member according to claim 1 or claim 2,
wherein the recess portion adopts a structure in which the suction portion and the non-suction portion are formed respectively into an elongated shape in the circumferential direction of the rotatable drum, the non-suction portions are plurally formed, and the suction portion and the non-suction portion are formed alternately in the width direction of the rotatable drum.

7. The method of producing an absorbent member according to claim 1 or claim 2,
wherein the recess portion adopts a structure in which the suction portion and the non-suction portion are formed respectively into an elongated shape in the width direction of the rotatable drum, the non-suction portions are plurally formed, and the suction portion and the non-suction portion are formed alternately in the circumferential direction of the rotatable drum.

8. The method of producing an absorbent member according to any one of claims 1 to 7,
wherein the bottom face of the non-suction portion is formed of an air-impermeable member which is placed on the porous plate; and
the air-impermeable member includes a side face portion having a linearly-shaped section in the thickness direction.

9. The method of producing an absorbent member according to any one of claims 1 to 8,
wherein the deposited aggregate is pressed in the pressing step between rolls each having a smooth surface or between a pair of emboss rolls in which a convex portion for embossing is provided to either roll or both rolls.

10. The method of producing an absorbent member according to any one of claims 1 to 9,
wherein the high density portion and the low density portion are pressed in the pressing step to have even apparent thickness.

## Patentansprüche

1. Verfahren zur Herstellung eines absorbierenden Elements, das aufweist:
einen Ablagerungsschritt zum Ablagern von Ausgangsmaterial des absorbierenden Elements, das zusammen mit einem Luftstrom durch Ansaugen zu einem Vertiefungsabschnitt zugeführt wird, der an einer Außenumfangsfläche einer drehbaren Trommel angeordnet ist; und
einen Pressschritt zum Pressen und Komprimieren einer abgelagerten Anhäufung, die von einem Inneren des Vertiefungsabschnitts abgegeben wird,
wobei der Vertiefungsabschnitt einen Saugabschnitt, der aus einer porigen Platte ausgebildet ist, um das Ansaugen von einer unteren Fläche durchzuführen, und einen Nichtsaugabschnitt mit einer luftundurchlässigen unteren Fläche hat, um das Ansaugen von der unteren Fläche nicht durchzuführen, umfasst, wobei die Tiefe des Nichtansaugabschnitts von der Außenumfangsfläche der drehbaren Trommel flacher als die Tiefe des Ansaugabschnitts von der Außenumfangsfläche der drehbaren Trommel ist;
wobei die abgelagerte Anhäufung in dem Ablagerungsschritt erhalten wird, indem das Ausgangsmaterial in den Vertiefungsabschnitt abgelagert wird; und
wobei in dem Pressschritt das absorbierende Element mit einem Abschnitt hoher Dichte und einem Abschnitt niedriger Dichte mit wechselseitig verschiedener Dichte erhalten wird, indem die von dem Vertiefungsabschnitt abgegebene abgelagerte Anhäufung gepresst wird;
wobei der Abschnitt niedriger Dichte zwischen den Abschnitten hoher Dichte eingefügt ist, wodurch die Abschnitte hoher Dichte in dem absorbierenden Element wechselseitig getrennt sind.

2. Verfahren zur Herstellung eines absorbierenden Elements nach Anspruch 1,
wobei das absorbierende Element ein absorbierendes Element ist, in dem ein Abschnitt, der dem Ansaugabschnitt entspricht, der Abschnitt hoher Dichte ist, und ein Abschnitt, der dem Nichtansaugabschnitt entspricht, der Abschnitt niedriger Dichte ist.

3. Verfahren zur Herstellung eines absorbierenden Elements nach Anspruch 1 oder 2,
wobei der Vertiefungsabschnitt mehrere Ansaugabschnitte umfasst, die jeweils eine rechteckige Fläche als den Ansaugabschnitt haben; und
die Ansaugabschnitte in einer Umfangsrichtung und einer Breitenrichtung der drehbaren Trommel mehrfach ausgebildet sind.

4. Verfahren zur Herstellung eines absorbierenden Elements nach Anspruch 3,
wobei ein Trennelement mit mehreren ersten Trennwänden, die sich in der Umfangsrichtung der drehbaren Trommel erstrecken, und mehreren zweiten Trennwänden, die sich in der Breitenrichtung der drehbaren Trommel erstrecken, im Inneren des Vertiefungsabschnitts angeordnet sind; und
die untere Fläche des Nichtansaugabschnitts aus dem Trennelement gebildet ist.

5. Verfahren zur Herstellung eines absorbierenden Elements nach Anspruch 4,
wobei die ersten Trennwände und die zweiten Trennwände verbunden sind.

6. Verfahren zur Herstellung eines absorbierenden Elements nach Anspruch 1 oder Anspruch 2,
wobei der Vertiefungsabschnitt eine Struktur verwendet, in welcher der Ansaugabschnitt und der Nichtansaugabschnitt in der Umfangsrichtung der drehbaren Trommel jeweils zu einer länglichen Form ausgebildet sind, die Nichtansaugabschnitte mehrfach ausgebildet sind, und der Ansaugabschnitt und der Nichtansaugabschnitt in der Breitenrichtung der drehbaren Trommel abwechselnd ausgebildet sind.

7. Verfahren zur Herstellung eines absorbierenden Elements nach Anspruch 1 oder Anspruch 2,
wobei der Vertiefungsabschnitt eine Struktur verwendet, in welcher der Ansaugabschnitt und der Nichtansaugabschnitt in der Breitenrichtung der drehbaren Trommel jeweils zu einer länglichen Form ausgebildet sind, die Nichtansaugabschnitte mehrfach ausgebildet sind, und der Ansaugabschnitt und der Nichtansaugabschnitt in der Umfangsrichtung der drehbaren Trommel abwechselnd ausgebildet sind.

8. Verfahren zur Herstellung eines absorbierenden Elements nach einem der Ansprüche 1 bis 7,
wobei die untere Fläche des Nichtansaugabschnitts aus einem luftundurchlässigen Element ausgebildet ist, das auf der porigen Platte angeordnet ist; und
das luftundurchlässige Element eine Seitenfläche mit einem linear geformten Schnitt in der Dickenrichtung umfasst.

9. Verfahren zur Herstellung eines absorbierenden Elements nach einem der Ansprüche 1 bis 8,
wobei die abgelagerte Anhäufung in dem Pressschritt zwischen Walzen, die jeweils eine glatte Oberfläche haben, oder zwischen einem Paar Prägewalzen, bei denen ein konvexer Abschnitt zum Prägen auf einer Walze oder beiden Walzen bereitgestellt ist, gepresst wird.

10. Verfahren zur Herstellung eines absorbierenden Elements nach einem der Ansprüche 1 bis 9,
wobei der Abschnitt hoher Dichte und der Abschnitt niedriger Dichte in dem Pressschritt gepresst werden, um eine gleichmäßige sichtbare Dicke zu haben.

## Revendications

1. Procédé pour produire un élément absorbant, comprenant :
une étape de dépôt consistant à déposer la matière première de l'élément absorbant alimentée conjointement avec un flux d'air jusqu'à une partie d'évidement qui est agencée au niveau d'une face circonférentielle externe d'un tambour rotatif par aspiration ; et
une étape de pression consistant à presser et à comprimer un agrégat déposé libéré d'un intérieur de la partie d'évidement,
dans lequel la partie d'évidement comprend une partie d'aspiration qui est formée d'une plaque poreuse pour réaliser l'aspiration à partir d'une face de fond et une partie sans aspiration ayant une face de fond imperméable à l'air pour ne pas réaliser l'aspiration à partir de la face de fond, alors que la profondeur de la partie sans aspiration à partir de la face circonférentielle externe du tambour rotatif est moins profonde que la profondeur de la partie d'aspiration à partir de la face circonférentielle externe du tambour rotatif ;
l'agrégat déposé est obtenu à l'étape de dépôt en déposant la matière première dans la partie d'évidement ; et
l'élément absorbant ayant une partie à densité élevée et une partie à faible densité avec une densité mutuellement différente, est obtenu à l'étape de pression en pressant l'agrégat déposé libéré de la partie d'évidement ;
dans lequel la partie à faible densité est intercalée entre les parties à densité élevée moyennant quoi les parties à densité élevée sont mutuellement séparées dans l'élément absorbant.

2. Procédé pour produire un élément absorbant selon la revendication 1,
dans lequel l'élément absorbant est un élément absorbant dans lequel une partie correspondant à la partie d'aspiration est la partie à densité élevée et une partie correspondant à la partie sans aspiration est la partie à faible densité.

3. Procédé pour produire un élément absorbant selon la revendication 1 ou 2,
dans lequel la partie d'évidement comprend une pluralité de parties d'aspiration ayant chacune une face de fond rectangulaire en tant que partie d'aspiration ; et
les parties d'aspiration sont formées en plusieurs exemplaires dans une direction circonférentielle et une direction de largeur du tambour rotatif.

4. Procédé pour produire un élément absorbant selon la revendication 3,
dans lequel un élément de séparation comprenant une pluralité de premières parois de séparation étendues dans la direction circonférentielle du tambour rotatif et une pluralité de secondes parois de séparation étendues dans la direction de la largeur du tambour rotatif, est agencé à l'intérieur de la partie d'évidement ; et
la face de fond de la partie sans aspiration est formée avec l'élément de séparation.

5. Procédé pour produire un élément absorbant selon la revendication 4,
dans lequel les premières parois de séparation et les secondes parois de séparation sont raccordées.

6. Procédé pour produire un élément absorbant selon la revendication 1 ou la revendication 2,
dans lequel la partie d'évidement adopte une structure dans laquelle la partie d'aspiration et la partie sans aspiration sont respectivement formées selon une forme allongée dans la direction circonférentielle du tambour rotatif, les parties sans aspiration sont formées en plusieurs exemplaires, et la partie d'aspiration et la partie sans aspiration sont formées de manière alternée dans la direction de largeur du tambour rotatif.

7. Procédé pour produire un élément absorbant selon la revendication 1 ou la revendication 2,
dans lequel la partie d'évidement adopte une structure dans laquelle la partie d'aspiration et la partie sans aspiration sont respectivement formées selon une forme allongée dans la direction de largeur du tambour rotatif, les parties sans aspiration sont formées en plusieurs exemplaires, et la partie d'aspiration et la partie sans aspiration sont formées de manière alternée dans la direction circonférentielle du tambour rotatif.

8. Procédé pour produire un élément absorbant selon l'une quelconque des revendications 1 à 7,
dans lequel la face de fond de la partie sans aspiration est formée avec un élément imperméable à l'air qui est placé sur la plaque poreuse ; et
l'élément imperméable à l'air comprend une partie de face latérale ayant une section de forme linéaire dans la direction de l'épaisseur.

9. Procédé pour produire un élément absorbant selon l'une quelconque des revendications 1 à 8,
dans lequel l'agrégat déposé est pressé à l'étape de pression entre des rouleaux ayant chacun une surface lisse ou entre une paire de rouleaux de gaufrage dans laquelle une partie convexe pour le gaufrage est prévue sur l'un ou l'autre des rouleaux ou sur les deux rouleaux.

10. Procédé pour produire un élément absorbant selon l'une quelconque des revendications 1 à 9,
dans lequel la partie à densité élevée et la partie à faible densité sont pressées à l'étape de pression pour avoir une épaisseur apparente régulière.
